(19) Europäisches Patentamt
European Patent Office
Office européen des brevets

(11) Publication number : 0 556 947 A1

# (12) EUROPEAN PATENT APPLICATION

(21) Application number : 93300208.1

(22) Date of filing : 14.01.93

(51) Int. Cl.[5] : A61K 31/00, A61K 31/55

(30) Priority : 21.01.92 US 823495

(43) Date of publication of application : 25.08.93 Bulletin 93/34

(84) Designated Contracting States : AT BE CH DE DK ES FR GB GR IE IT LI LU NL PT SE

(71) Applicant : MERCK & CO. INC.
126, East Lincoln Avenue P.O. Box 2000
Rahway New Jersey 07065-0900 (US)

(72) Inventor : Mallorga, Pierre
304, Abbey lane
Lansdale,PA 19446 (US)

(74) Representative : Thompson, John Dr. et al
Merck & Co., Inc. European Patent Department Terlings Park Eastwick Road
Harlow, Essex CM20 2QR (GB)

## (54) Use of M1-selective antimuscarinic pyridobenzodiazepinones for the treatment and prevention of axial myopia.

(57) Pyridobenzodiazepinones of the formula :

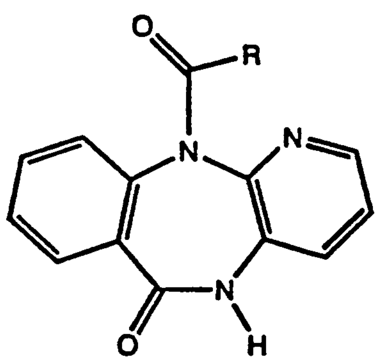

wherein R is piperidine substituted by $C_{1-5}$ alkyl, are ocularly administered to animals to prevent abnormal increases in eye axial length and thereby arrest the progression of axial myopia. The compounds are pharmacologically characterized in that they selectively antagonize the binding of acetylcholine to $M_1$-type muscarine receptors.

EP 0 556 947 A1

BACKGROUND OF THE INVENTION

This invention relates to control of ocular development in general and, more particularly, to the treatment of the eye to prevent and/or arrest the development of myopia (nearsightedness).

Myopia afflicts approximately 25% of the world's population. In particular, myopia afflicts 15% to 75% of the youth of the world, depending upon race, geographic distribution and level of education. Approximately one-half of all cases are classifiable as axial myopia, where there is an elongation of the eye along the visual axis.

At birth, the human eye is about two-thirds of the size of an adult eye. This is relatively short in the direction of the visual axis, or axial, direction. As a result, children tend to be farsighted. During childhood, as the eye grows, there is a compensatory mechanism governing changes in the structure of the cornea and lens in response to increasing ocular length during childhood growth. Often the process is virtually perfect and no correction is needed for sharp vision at a distance. However, when regulatory mechanisms fail, the eye tends to axially lengthen. As a result, distant images focus in front of the plane of the retina and the result for the patient is axial myopia.

Myopia is not a trivial maldevelopment of the eye. In its pathologic form, the sclera continues to grow as the retina stretches and degenerates resulting in permanent blindness. The surgical therapies attempted for this condition are drastic and often unsuccessful.

Even in its milder forms, myopia has been shown to have an effect on the self-image of a child during the critical state of his development. Children who wear glasses, justly or not, are considered bookish, introverted, and nonathletic (Curtin, 1985). Because of these childhood memories, a parent that is myopic is often particularly eager to inquire of the doctor about a therapy to limit the development of the myopia when it has been diagnosed in his child.

Furthermore, the optical corrections which exist for myopia are neither ideal nor risk-free. There are roughly 24 million contact lens wearers in the United States, and the number is expected to double in the next five years. No matter how well fitted or cared for, complications of contact lens wear range from allergic reactions to permanent loss of vision due to corneal ulceration. The latter problem was so serious that the Food and Drug Administration (FDA) reduced the recommended wearing time for extended wear contact lenses from 30 days to "one to seven days". In 1988, over 20,000 injuries related to contact lens wear were treated and reported by hospital emergency rooms.

While eyeglasses eliminate most of the medical risks listed above, they are not an acceptable option as evidenced by the contact lens wearers who tolerate the frustration of contact lens wear. As a result, large efforts have been devoted to the correction of myopia by corneal surgery using conventional knives or excimer lasers. Neither of these therapies are easily reversed or sufficiently predictable in their results. Despite the risk of permanent visual impairment associated with these surgical therapies, thousands of patients have undergone radial keratotomy in the United States.

While the optical and surgical correction of myopia corrects the refractive state of the eye, these therapies do not address the abnormal elongation of the eye during development. Surveys indicate that even low degrees of myopia (-1.00 to -5.00 diopters) predispose patients to the development of glaucoma (Perkins, 1982). In myopia, the retina is stretched and thinned, which predisposes the eye to retinal detachment. Consequently, several studies have documented myopia as a risk factor for the development of retinal detachment. All of the above highlight the need for a pharmacologic therapy to address the developmental abnormality of myopia.

Previously available therapies that relied on administration of drugs began with the use of cycloplegic agents. Cycloplegics are topically administered drugs that relax the ciliary muscle of the eye, which is the muscle that focuses the eye by controlling lens dimensions.

The classic cycloplegic drug is the belladonna alkaloid atropine, available for over a century. Atropine is a long-acting non-specific antimuscarinic agent that antagonizes the action of the neurotransmitter acetylcholine (ACh) at autonomic effector cells innervated by postganglionic cholinergic nerves of the parasympathetic nervous system.

In 1971 Bedrossian completed a two-year crossover study in 75 children with myopia. During the first year, the right eye was treated with 1.0% atropine applied topically at bedtime. The left eye was treated during the second year. As shown by Table 1, the progression of the myopia was halted in the treated eye (See Table 1).

Table 1. Effect of 1.0% Atropine Upon Myopia (Bedrossian, 1971)

[mean change in refraction in diopters]
[+ for hyperopia, - for myopia]

| | Right Eye | Left Eye |
|---|---|---|
| Year 1 | +0.20 | -0.85 |
| Year 1 | -1.05 | +0.17 |

The results were significant at less than the 1% level.

A later study confirmed the findings of the first study and indicated that the greatest effect of atropine upon the slowing of the rate of progression of myopia was seen in children under nine years of age (See Table 2).

Table 2. Progression of Myopia (Brodstein, 1984)

[progression in diopters per year]
[+ for hyperopia, - for myopia]

| Age of Onset of Therapy | Therapy with 1.0% Atropine | Control |
|---|---|---|
| < 9 years | -0.23 | -0.69 |
| 9-12 years | -0.24 | -0.48 |
| > 13 years | -0.20 | -0.23 |

Atropine has not been widely used for slowing the progression of myopia due to drug effects including glare from pupillary dilation, and inhibition of ocular focusing that impairs near visual work like reading.In addition to the discomfort to the patient, there is evidence that long-term use of atropine or other long-term cycloplegics can harm the retina when it is exposed to bright light.

Recently, selective-acting antimuscarinic agents were discovered to be useful in myopia therapy. This discovery was made possible by a new body of substantial evidence to link the posterior part of the eye, specifically the image quality regions of the retina and hence an extension of the nervous system, to the postnatal regulation of ocular growth. There is significant evidence of myopia resulting in an eye that is subjected to retinal image degradation. It has been shown that axial myopia can be experimentally induced, in either birds or primates, in an eye in which the retina is deprived of formed images, e.g., by suturing the eyelids or wearing an image diffusing goggle. The experimental myopia induced in birds or primates such as monkeys mimics the common axial myopia of humans.

Thus, the phenomenon of an animal's vision process apparently contributes to the feedback mechanism by which postnatal ocular growth is normally regulated and refractive error is determined. This indicates that this mechanism is neural and likely originates in the retina. R. A. Stone, et al. have found a method of controlling the abnormal postnatal growth of the eye of a maturing animal, which comprises controlling the presence of a neurochemical, its agonist or antagonist, which neurochemical is found to be changed under conditions during maturation leading to abnormal axial length. Therein it is disclosed that in experimental animals such as chicks or monkeys subjected to ocular image deprivation ordinarily leading to the development of myopia, the metabolism of certain retinal neurochemicals is altered leading to changes in retinal concentrations thereof. Specifically, retinal concentrations of dopamine were found to be reduced during such image deprivation and the ocular administration of a dopamine-related agent, e.g., apOmorphine, a dopamine agonist, was found to inhibit or actually prevent the axial enlargement of the eye under conditions ordinarily leading to such enlargement.

There have been recent advances made in the understanding of the cholinergic nervous system. Cholinergic receptors are proteins embedded in the wall of a cell that respond to the chemical acetylcholine. These receptors are broadly broken down into nicotinic and muscarinic receptors. In this respect, it is now known that the muscarinic receptors are not all of one type. Recent findings show that there are at least five, if not more, types of cholinergic muscarinic receptors (types $M_1$ through M5). Type $M_1$ receptors are those present in abundance and thought to be enriched in the brain neural tissue and neural ganglia. Other receptors are concentrated in other tissues, such as in heart, smooth muscle tissue, or glands. While many pharmacological agents

interacting with muscarinic receptors influenceseveral types, some agents are known to have a major effect on a single type of receptor with relative selectivity and other agents can have a relatively selective effect on a different single receptor. Still other agents may have a significant effect on more than one or even all types of receptors. It is known, for example, that pirenzepine, (Gastrozepin, LS 519) 5,11-Dihydro-11-[4-methyl-1-piperazinyl) acetyl]-6H-pyrido[2,3-b][1,4]benzodiazepin-6-one, and its dihydrochloride, are anticholinergic, antimuscarinic, and relatively selective for $M_1$ receptors. It is also known that 4-DAMP (4-diphenylacetoxy -N-methylpiperadine methiodide) is - relatively selective antagonist for smooth muscle (ordinarily called $M_3$ type but variously called type $M_2$ or $M_3$, as the current classification of receptors is in flux). In contrast, it is believed that atropine, discussed above as a cycloplegic agent, is a non-specific antagonist for all types of cholinergic muscarinic receptors.

Pirenzepine, being primarily an $M_1$ antagonist, inhibits axial elongation, but is far less effective at pupil dilation than atropine or another cycloplegic agent. This makes it possible to suppress the development of myopia without dilating the pupil and paralyzing the accommodation activity of the ciliary body.

Pirenzepine, however, has a disadvantage. The administration of a drug topically into the eye of a developing child for a long period of time makes it desireable to have a minimal likelihood of sensitization of the eye. Pirenzepine tests positive in sensitization assays. There is therefore a need in the art for a selective antimuscarinic agent that can be used to treat myopia, which will not cause effects on pupil size or accommodation of the ciliary body, and which will be less likely to cause sensitization than pirenzepine.

## SUMMARY OF THE INVENTION

The invention is a method of using a compound of the formula:

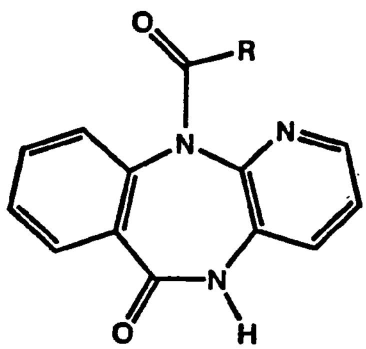

I

and the pharmaceutically acceptable salts thereof, where R is piperidine substitued by $C_{1-5}$ alkyl, in the therapy of myopia by ocular administration of such a compound, which will prevent abnormal increases in the axial lenght of the eye.

Specifically, the compounds rispenzepine (ulenzepine)

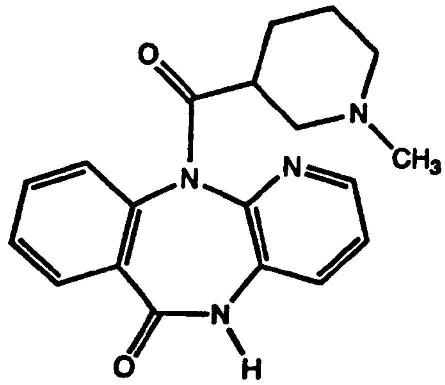

and nuvenzepine

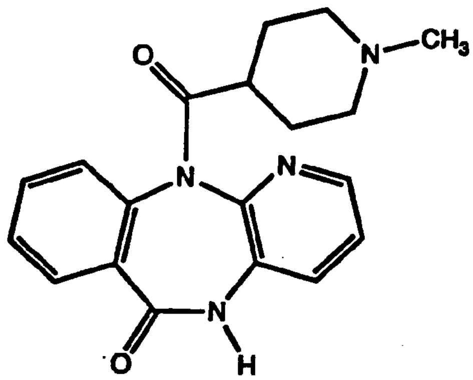

can be used in the method of the present invention, by their ocular administration to prevent abnormal increases in the axial length of the eye and thus prevent progression of axial myopia.

DETAILED DESCRIPTION OF THE INVENTION

Compounds of formula I are prepared starting from compounds of formula II, described in J. Med. Chem. 6, 255, 1963; Bull. Soc. Chim. Fran. 7, 2316, 1966; and German Pat. No. 1,179,943, by acylation with the acyl chlorides of formula III, according to the following scheme:

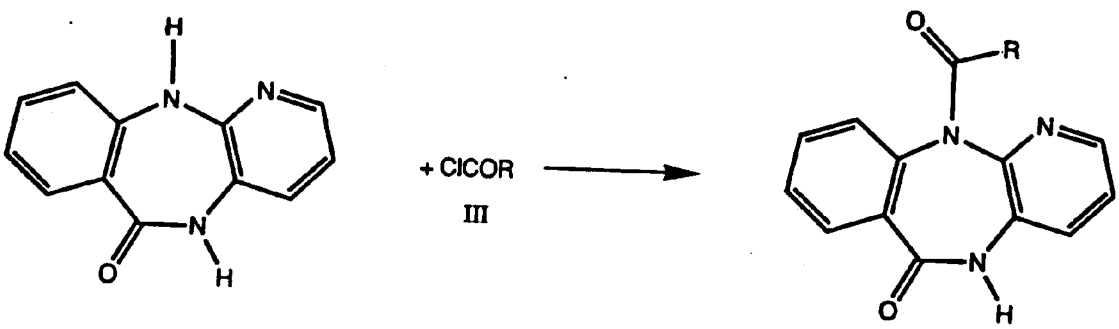


II

where R is defined as before, that is piperidine substituted by $C_{1-5}$ alkyl. The reaction is carried out in polar solvents such as dimethylformamide and dimethylsulfoxide in the presence of bases such as triethylamine, alkaline hydroxides and alkaline carbonates at temperatures ranging from room temperature to 150°C, with reaction times ranging from 2 to 24 hours.

Representative pharmaceutically acceptable salts include, for example, hydrochloride and maleate. Salts are generally prepared as acid addition salts by combining the core compound with one-to-three equivalents of an appropriate acid in an inert solvent. The salt is then recovered by solvent evaporation or by filtration if the salt precipitates spontaneously, or by precipitation using a co-solvent or a non-polar co-solvent followed by filtration.

The term "pharmacologically effective amount" shall mean that amount of a drug or pharmaceutical agent that will elicit the biological or medical response of a tissue, system or organ that is being sought by a veterinarian or physician.

The term "alkyl" shall mean linear straight or branched chain alkane or alkene.

The term "substituted" shall be deemed to include multiple degrees of substitution by a named substituent.

The compounds used in the method of the invention can be prepared readily according to the following detailed examples using readily available starting materials, reagents and conventional synthesis procedures. In these reactions, it is also possible to make use of variants which are themselves known to those of ordinary skill in this art, but which are not mentioned in greater detail. Additional background information is taught in U.S. Patent No. 4,556,653, the entire disclosure of which is incorporated herein by reference.

EXAMPLE 1

11-(1-Methylpiperidin-3-carbonyl)-5,11-dihydro-6H -pyrido[2,3-b][1,4]benzodiazepin-6-one

The chloride of 1-methylpiperidine-3-carboxylic acid hydrochloride (4.7g) is added to a solution of 5,11-dihydro-6H-pyrido[2,3-b][1,4] benzodiazepin-6-one (5g) and triethylamine (7.5ml) in dimethylformamide (200ml). The reaction mixture is heated to 90°C for 24 hours.

EXAMPLE 2

11-(1-Methylpiperidin-4-carbonyl)-5,11-dihydro-6H -pyrido[2,3-b][1,4]benzodiazepin-6-one

The chloride of 1-methylpiperidine-4-carboxylic acid hydrochloride (4.7g) was added to a solution of 5,11-dihydro-6H-pyrido[2,-3b][1,4] benzodiazepin-6-one (5g) and triethylamine (7.5ml) in dimethylformamide (200ml). The reaction mixture was heated to 90°C for 24 hours.

The solvent was evaporated under vacuum and the residue was dissolved in 10% acetic acid (150ml). The solution was repeatedly washed with methylene chloride, decolored with charcoal, alkalinized to pH 8 with a saturated solution of sodium bicarbonate and extracted with methylene chloride (100ml x 5). The collected organic extracts were dried on sodium sulphate and evaporated. The residue, crystallized from ethanol-ethylether, yielded 2.4g (30%) of 11-(1-methylpiperidin-4-carbonyl)-5,11-dihydro-6H-pyrido (2,3-b][1,4]benzodiazepin-6-one melting at 265°-267°C dec.

The muscarinic agents for use in this invention are those relatively selective in blocking the $M_1$ type receptors and which are relatively less selective for the type $M_3$ smooth muscle receptors. Relative affinities for $M_1$-$M_5$ receptors were determined using the following assay.

EXAMPLE 3

Three radioligand binding assays were established in order to determine the affinities of muscarinic antagonists for $M_1$, $M_2$ and $M_3$ muscarinic receptor subtypes. $M_1$ receptors in rat cerebral cortex were labeled with $^3$H-pirenzepine, $^3$H-AFDX-384 labeled $M_2$ receptors in rat heart and $M_3$ receptors in rabbit iris + ciliary body (minus ciliary processes which contain $M_1$ receptors) were labeled with $^3$H-QNB. Scatchard analysis revealed that one population of binding sites was labeled in each tissue and the following Kd and Bmax values were obtained: cortex (2.36 nM, 1781 fmol/mg protein), heart (5.77 nM, 164 fmol/mg protein) and iris + ciliary body (24.5 pM and 210 fmol/mg protein). Ki values in the three binding assays were obtained for atropine, rispeniepine, pirenzepine and nuvenzepine. The most potent of the agents at $M_1$ binding sites was atropine with a Ki value of 0.13 nM. Pirenzepine, nuvenzepine and rispenzepine possessed Ki values of 4.87 nM, 1.50 nM and 2.61 nM, respectively. Atropine was the most potent of the antagonists at M2 receptors possessing a Ki of 0.82 nM while pirenzepine (532 nM), nuvenzepine (248 nM) and rispenzepine (226 nM) were much less effective. Atropine was also the most potent (0.37 nM) at $M_3$ binding sites being followed by nuvenzepine (29.7 nM), pirenzepine (37.2 nM) and rispenzepine (38.9 nm). In terms of their ability to distinguish between $M_1$ and $M_3$ receptors, atropine was essentially devoid of selectivity while pirenzepine, nuvenzepine and rispenzepine were respectively 8-, 20- and 15-fold more selective for $M_1$ than for $M_3$ receptors.

Affinity of Several Muscarinic Antagonists for $M_1$, $M_2$ and $M_3$ Muscarinic Receptors

| Drug | $M_1$ Ki | $M_1$ nH | $M_2$ Ki | $M_2$ nH | $M_3$ Ki | $M_3$ nH |
|---|---|---|---|---|---|---|
| Atropine | 0.13 ± 0.2 | 1.5 | 0.82 ± 0.23 | 1.05 | 0.37 ± 0.21 | 1.1 |
| Pirenzepine | 4.87 ± 2.55 | 1.0 | 532 ± 93 | 0.90 | 37.2 ± 3.80 | 0.72 |
| Rispenzepine | 2.61 ± 0.39 | 0.9 | 226 ± 54 | 1.00 | 38.9 ± 2.90 | 0.84 |
| Nuvenzepine | 1.50 ± 0.53 | 0.9 | 248 ± 11 | 1.03 | 29.7 ± 3.00 | 0.80 |

Ki values = The mean of 3-7 separate determinations±SEM and are expressed in nM.

nH = Hill Coefficient.

$M_1$ = $^3H$ Pirenzepine binding, rat cerebral cortex.

$M_2$ = $^3H$ AFDX-384 binding, rat heart.

$M_3$ = $^3H$-QNB binding, rabbit iris + ciliary body (minus ciliary processes).

Alternatively, the affinity and relative affinity of muscarinic antagonists for $M_1$-$M_5$ receptors can be determined by other means known in the art. For example, see Buckley, et al., Molecular Pharmacology, 35: 469-476 (1989) or Dorje, et al., J. Pharmacol. Exp. Ther. 256:727-733 (1991) for detailed descriptions of techniques known in the art for determining the antagonist binding properties of five cloned human muscarinic receptors. Similarly, there are other ways in which to accomplish functional studies to measure $M_1$ sensitivity. For instance, one popular method at present is to use vas deferens of the guinea pig which has an $M_1$ sensitivity. First it is set up so that its tension is measured and a known stimulator such as the M1 agonist McNeil A343

is given to change tension by a predictable amount. Under this condition, the predicted effect of the agonist is first carefully plotted and then the degree to which one or another antagonist blocks this agonist effect is measured.

An in vivo animal model of myopia is available for screening, which is described as follows.

Form-deprivation myopia is induced in day-old White Leghorn chicks under aseptic conditions and other anesthesia by eyelid suture to one eye. The chicks are maintained on a 12-hour light:dark cycle. The sutured eyes are treated with nuvenzepine, rispenzepine and pirenzepine and saline solution as a control. Drug is injected daily subconjunctivally during the light cycle. At two weeks of age the animals are sacrificed and axial and equatorial dimensions of unfixed eyes are measured with vernier calipers independently by two observers. Rispenzepine and nuvenzepine, because of their somewhat greater potency at the $M_1$ receptor relative to pirenzepine, would be expected to be at least as effective as pirenzepine in myopia therapy.

## EXAMPLE 4

The guinea pig maximization test is required by the U.S. Food and Drug Administration of all drugs intended for ophthalmic administration. The test population for nuvenzepine and rispenzepine versus pirenzepine consisted of at least nine female Dunkin Hartley albino guinea pigs between 300 and 500g, with 10 control animals. In Phase I, which is the induction phase, on day 1 two intradermal injections at the highest generally tolerated concentration of the test material were administered in the shaved intercapsular area (O.1ml of the test agent in a 50:50 mixture of Freund's complete adjuvant and distilled water). On day 7, approximately 400mg of a mixture of 10% sodium laurel sulfate in petrolatum was applied to the shaved intercapsular area. On day 8, 400mg of a mixture of the test compound in petrolatum was applied, on a 2 x 4 cm piece of Whatman paper to the shaved intercapsular area, along with Blenderm tape.

In Phase II, which is the challenge phase, on day 22, 200mg of petrolatum was applied to the shaved right flank, while 200mg of test compound at the highest non-irritant concentration (micronized in petrolatum) was applied to the shaved left flank, with each application being occluded for 24 hours.

Readings were taken on days 24 and 25, with grades assigned as follows:

0 = no reaction
1 = scattered mild redness
2 = moderate and diffused redness
3= intense redness and swelling

Nuvenzepine and rispenzepine both showed 0, no reaction in any test animal. pirenzepine, however, showed a grade of "1" in six of the animals and a grade of "2" in two of the animals, out of a total population of nine animals.

Diagnosis and treatment, using the method of the invention, is well known to those of ordinary skill in the art.

The refractive state of the eye is easily determined even in newborn infants by the use of a retinoscope, a handheld instrument. This objective measurement is performed after cycloplegia of the eye has been obtained by topical administration of cyclopentolate or atropine. Furthermore, the axial length of the eye can easily be measured using ultrasonography.

Myopia becomes manifest as children reach the age of 7 to 12 years. Once identified, the myopia usually progresses until the eye completes, development after the age of 14.

Once identified, the progression of myopia can be documented by examination every 6 to 12 months. Risk factors for the development of high degrees of myopia include race and family history.

Once the myopia has been documented as progressing, pharmacologic therapy could be initiated to reduce the rate of progression. Therapy would be continued until the ocular development is completed. For most children, therapy would be initiated between the ages of 7 and 10 years and discontinued around the age of 14 years.

Therapy to inhibit axial-elongation myopia during maturation can be administered by the use of the agent in eye drops. Indeed, in the vast majority of cases, treatment agents are administered to human eyes by the application of eye drops. Eye drops are typically made up at a concentration of active agent between about 0.5 and 2 percent in the ophthalmic medium. A 1% solution of rispenzepine or nuvenzepine in water would be a likely concentration for clinical use. Some constraints in formulation may exist having to do with pH and preservative. A pH of about 6.5 is expected to be acceptable as an ophthalmic drop and practical in terms of known solubility and stability of pyridobenzodiazepinones. Since pirenzepine is known to form very acidic solutions in physiological saline, treatment with known compatible bases to bring the pH up to about 4.5 to 7.5 (preferably 6 or 6.5) is recommended. Phosphate buffering is also common for eye drops and is compatible with rispenzepine and nuvenzepine. A common regimen for application of eye drops is two to three times a day spaced

evenly throughout waking hours. More effective agents may require fewer applications or enable the use of more dilute solutions. Alternatively, ointments and solid inserts are now coming into increased use in clinical practice. They avoid problems of drug decomposition while delivering a defined amount of drug. It is, of course, also possible to administer the above-described active agents in therapeutically effective amounts and dosages in pills, capsults, or other preparations of systemic administration.

In experiments in animals, such as those mentioned hereinabove in which axial myopia has been experimentally induced by depriving the retina of formed images, it has been noted by others in primates that amblyopia was also experimentally and coincidentally induced. Amblyopia is evidenced by poor visual acuity in the eye resulting in poor visual performance. Normally, visual acuity improves during maturation. It is known that amblyopia may occur in humans from unknown causes or as part of strabismus. It is possible that administration of therapeutically effective amounts and dosages of the muscarinic antagonists relatively selective in blocking the $M_1$ cholinergic receptors, but less selective in blocking cholinergic receptors in smooth muscle cells, e.g. rispenzepine and nuvenzepine, might prevent or inhibit the development of permanent or persistent amblyopia in maturing humans with decreased likelihood of sensitization of the eye. It is also possible that humans who have already developed amblyopia from other or even unknown causes might be aided by similar therapeutic treatment with the aforementioned agents.

In addition to rispenzepine and nuvenzepine, other selective antimuscarinic pyridobenzodiazepinones within the scope of the invention include the following.

N,N-Dime thyl-4-[[5,11-dihydro-6-oxo-6H-pyrido [2,3-b][1m4]benzodiazepin-11-yl]carbonyl] piperidinium iodide

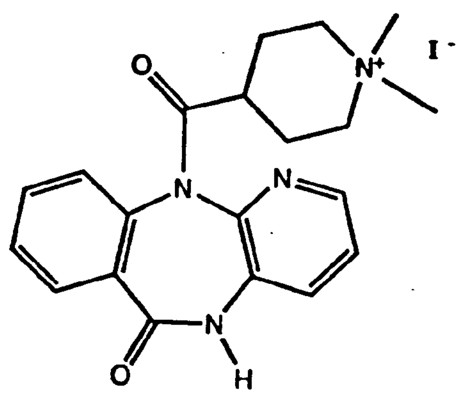

11-[(1-ethylpiperidin-4-yl)carbonyl]-6,11-dihydro -5-oxo-5H-pyrido[(1,2-b][1,5]benzodiazepine

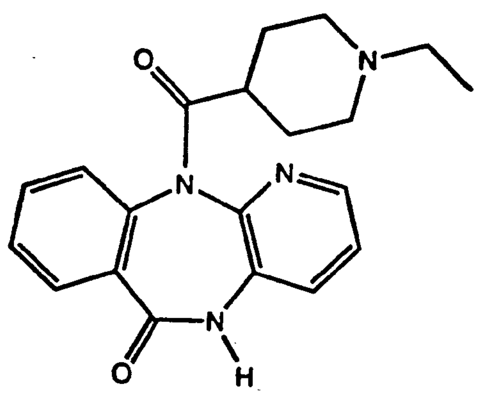

## Claims

1. The use, for the manufacture of an ocularly administrable medicament for preventing abnormal increase in eye axial length in an animal in need thereof, of a compound of formula:

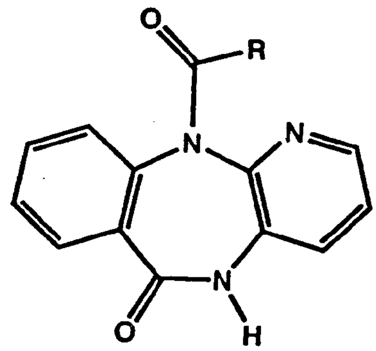

wherein R is piperidine substituted by $C_{1-5}$ alkyl.

2. The use, for the manufacture of an ocularly administrable medicament for preventing abnormal increase in eye axial length in an animal in need thereof, of a compound of formula:

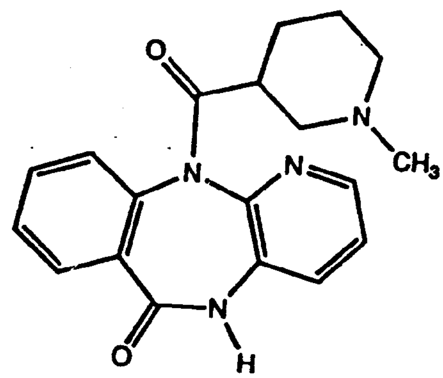

3. The use, for the manufacture of an ocularly administrable medicament for preventing abnormal increase in eye axial length in an animal in need thereof, of a compound of formula:

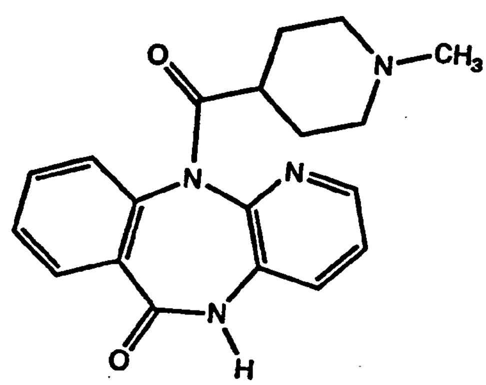

4. The use, for the manufacture of an ocularly administrable medicament for preventing progression of axial myopia in an animal in need thereof, of a compound of formula:

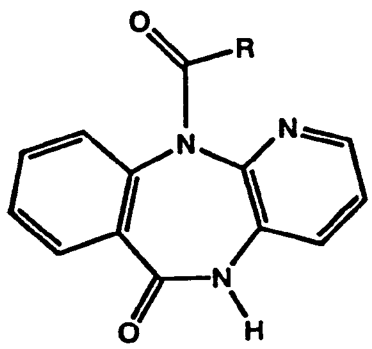

where R is piperidine substituted by $C_{1-5}$ alkyl.

5. The use, for the manufacture of an ocularly administrable medicament for preventing progression of axial myopia in an animal in need thereof, of a compound of formula:

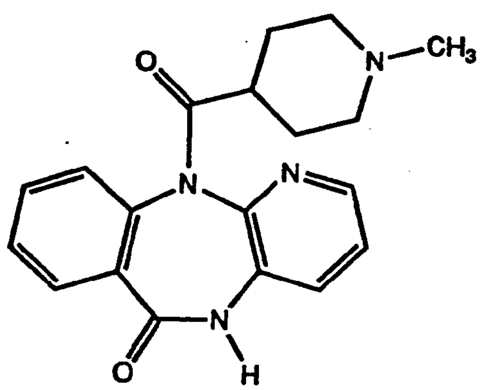

6. The use, for the manufacture of an ocularly administrable medicament for preventing progression of axial myopia in an animal in need thereof, of a compound of formula:

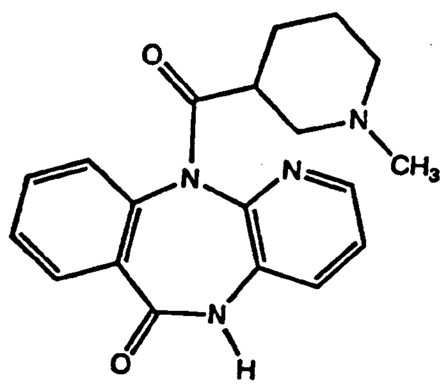

European Patent Office

**PARTIAL EUROPEAN SEARCH REPORT**
which under Rule 45 of the European Patent Convention shall be considered, for the purposes of subsequent proceedings, as the European search report

Application Number
EP 93 30 0208
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| Y | WO-A-9 015 604 (THE TRUSTEES OF THE UNIVERSITY OF PENSYLVANIA)<br>27 December 1990<br>* the whole document *<br>--- | 1-6 | A61K31/00<br>A61K31/55 |
| Y | EXPERIMENTAL EYE RESEARCH<br>vol. 52, no. 6, June 1991,<br>pages 755 - 758<br>STONE R.A. ET AL 'MUSCARINIC ANTAGONIST EFFECTS ON CHICK MYOPIA'<br>* the whole document *<br>--- | 1-6 | |
| Y | EUROPEAN JOURNAL OF PHARMACOLOGY<br>vol. 179, no. 1-2, 10 April 1990,<br>pages 89 - 96<br>BAROCELLI E. ET AL 'EFFECTS OF TWO NEW PIRENZEPINE ANALOGS ON THE CONTRACTILE RESPONSE OF THE GUINEA-PIG OESOPHAGEAL MUSCULARIS MUCOSAE TO ACETYLCHOLINE, BETHANECOL, HISTAMINE AND HIGH POTASSIUM'<br>* page 90, column 1; figure 1 *<br>* page 94, column 2, line 17 - page 95, column 1, line 12 *<br>---<br>-/-- | 1-6 | |

TECHNICAL FIELDS SEARCHED (Int. Cl. 5)
A61K

## INCOMPLETE SEARCH

The Search Division considers that the present European patent application does not comply with the provisions of the European Patent Convention to such an extent that it is not possible to carry out a meaningful search into the state of the art on the basis of some of the claims
Claims searched completely :
Claims searched incompletely :
Claims not searched :
Reason for the limitation of the search:

see sheet C

| Place of search | Date of completion of the search | Examiner |
|---|---|---|
| THE HAGUE | 06 MAY 1993 | MAIR J. |

CATEGORY OF CITED DOCUMENTS
X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04E07)

European Patent Office

# PARTIAL EUROPEAN SEARCH REPORT

Application Number
EP 93 30 0208
Page 2

| DOCUMENTS CONSIDERED TO BE RELEVANT | | | CLASSIFICATION OF THE APPLICATION (Int. Cl. 5) |
|---|---|---|---|
| Category | Citation of document with indication, where appropriate, of relevant passages | Relevant to claim | |
| D,Y | EP-A-0 125 607 (DOMPE\` FARMACEUTICI SPA) 21 November 1984<br>* page 18examples 43,46; table II *<br>* page 17example 38; table I *<br>* page 21, line 27 - page 23, line 12 *<br>----- | 1-6 | |
| | | | TECHNICAL FIELDS SEARCHED (Int. Cl. 5) |

EPO FORM 1503 03.82 (P04E10)

EP 93 30 0208 -C-

INCOMPLETE SEARCH

Claims searched completely : 2,3,5,6
Claims searched incompletely : 1,4

Reason : In view of the large number of compounds which are theoretically defined by the Markush formula of claim 1 and the fact that some of the compounds mentioned in the description are not carried by the formula, the search has been directed mainly towards the compounds specifically claimed, for reasons of economy.